# EUROPEAN PATENT APPLICATION

(11) **EP 4 840 401 A1**
(43) Date of publication of application: **18.11.2026**
(21) Application number: 24917194.3
(22) Date of filing: 11.01.2024
(51) Int. Cl.: A61B 6/03

(54) **LESION IDENTIFICATION METHOD AND LESION IDENTIFICATION PROGRAM**

(71) Applicant: FUJITSU LIMITED, Kawasaki-shi, Kanagawa 211-8588 (JP)
(72) Inventor: MIYAZAKI, Nobuhiro, Kawasaki-shi, Kanagawa 211-8588 (JP); TAKEBE, Hiroaki, Kawasaki-shi, Kanagawa 211-8588 (JP); BABA, Kozo, Kawasaki-shi, Kanagawa 211-8588 (JP); BABA, Takayuki, Kawasaki-shi, Kanagawa 211-8588 (JP)
(74) Representative: Hoffmann Eitle
(86) International application number: PCT/JP2024/000331
(87) International publication number: WO 2025/150130

(57) **Abstract**

A region of a lesion in a medical image is identified with high accuracy.

A lesion identification apparatus (1) obtains, for each image block included in a tomographic image (2), a first probability that the image block is a region of a specific lesion, by inputting the tomographic image (2) into a machine learning model (4a) configured to identify, in units of image blocks obtained by dividing an input tomographic image into a fixed size, whether each image block is a region of the specific lesion, generates, based on the obtained first probabilities, probability data (3) including a second probability assigned to each pixel included in the tomographic image (2), and identify, for each pixel of the tomographic image (2), whether the pixel is a region of the specific lesion, by inputting the tomographic image (2) and the probability data (3) into a machine learning model (4b) configured to identify, in units of pixels included in the input tomographic image, whether each pixel is a region of the specific lesion.

## Description

### Technical Field

The embodiments discussed herein relate to a lesion identification method and a lesion identification program.

### Background Art

Medical images obtained by computed tomography (CT), magnetic resonance imaging (MRI), and the like are widely used for diagnosis of various diseases. In image diagnosis using such medical images, doctors need to interpret a large number of images, which imposes a heavy burden on the doctors. Therefore, there is a demand for techniques that use computers to assist doctors in making diagnosis.

One example of such techniques is a technique that processes medical images using a trained model (a machine learning model) generated through machine learning. For example, a method has been proposed in which a segmentation model trained through a machine learning process is used to segment a target image to obtain a target region image including a target object region.

In addition, as an example of image recognition techniques, an image processing apparatus has been proposed in which an attribute score map representing an attribute of each region of an input image is generated for each attribute based on a result of processing the input image using a hierarchical neural network, the attribute score maps are integrated to generate a recognition result for a recognition target, and the recognition result is output.

### Citation List

### Patent Literature

PTL1: International Publication Pamphlet No. WO 2021/202204
PTL2: Japanese Laid-open Patent Publication No. 2022-173399

### Summary of Invention

### Technical Problem

Incidentally, there is a technique for identifying, using a machine learning model, whether a region in a medical image is a lesion region. For example, there is a machine learning model that divides a medical image into image blocks of a fixed size and identifies, in units of image blocks, whether each image block is a lesion. While this machine learning model is able to identify lesions with few missed detections, it may misidentify non-lesion regions as lesions.

As another example, there is also a machine learning model that identifies, upon receiving a single medical image, whether each pixel of the medical image is a lesion. Since this machine learning model identifies lesions based on image features of the entire medical image, such as the shape of a lesion and its positional relationship with an organ, it is less likely to misidentify non-lesion regions as lesions, as compared with the above-described machine learning model. However, it is more likely to misidentify lesion regions as non-lesions, as compared with the above-described machine learning model.

As described above, each machine learning model for identifying lesions has advantages and disadvantages, and there is no highly accurate machine learning model with few disadvantages.

In one aspect, an object of the present disclosure is to provide a lesion identification method and a lesion identification program capable of identifying a lesion region in a medical image with high accuracy.

### Solution to Problem

According to an aspect, there is provided a lesion identification method, in which a computer: acquires a first tomographic image obtained by imaging an inside of a first human body; obtains, for each first image block included in the first tomographic image, a first probability that the first image block is a region of a specific lesion, by inputting the first tomographic image into a first machine learning model, the first machine learning model being configured to identify, in units of first image blocks obtained by dividing an input tomographic image into a predetermined size, whether each of the first image blocks of the input tomographic image is a region of the specific lesion; generates, based on the obtained first probability, first probability data including a second probability for each pixel included in the first tomographic image; and identifies, for each pixel included in the first tomographic image, whether the pixel is a region of the specific lesion, by inputting the first tomographic image and the first probability data into a second machine learning model, the second machine learning model being configured to identify, in units of pixels included in the input tomographic image, whether each of the pixels included in the input tomographic image is a region of the specific lesion.

In addition, according to an aspect, there is provided a lesion identification program, which causes a computer to perform the same processing as described above for the lesion identification method.

### Advantageous Effects of Invention

In one aspect, it is possible to identify a lesion region in a medical image with high accuracy.

The foregoing and other objects, features, and advantages of the present disclosure will become apparent from the following description of preferred embodiments, when read together with the accompany drawings.

### Brief Description of Drawings

[FIG. 1] FIG. 1 illustrates a configuration example and a processing example of a lesion identification apparatus according to a first embodiment.
[FIG. 2] FIG. 2 illustrates a configuration example of a diagnosis support system according to a second embodiment.
[FIG. 3] FIG. 3 illustrates an example of a hardware configuration of a lesion identification apparatus.
[FIG. 4] FIG. 4 illustrates a first example of a lesion identification model.
[FIG. 5] FIG. 5 illustrates a second example of the lesion identification model.
[FIG. 6] FIG. 6 illustrates a lesion identification process according to the second embodiment.
[FIG. 7] FIG. 7 illustrates a configuration example of processing functions included in each apparatus of the diagnosis support system.
[FIG. 8] FIG. 8 illustrates an example of data stored in a training data storage unit.
[FIG. 9] FIG. 9 illustrates an example of data stored in a model data storage unit.
[FIG. 10] FIG. 10 is a flowchart illustrating an example of a training process performed by a training apparatus.
[FIG. 11] FIG. 11 is a flowchart illustrating an example of a score map generation process.
[FIG. 12] FIG. 12 is a flowchart illustrating an example of a lesion identification process performed by a lesion identification apparatus.
[FIG. 13] FIG. 13 illustrates an example of a screen that displays a lesion identification result.
[FIG. 14] FIG. 14 illustrates a lesion identification process according to a third embodiment.
[FIG. 15] FIG. 15 is a flowchart illustrating an example of a training process according to the third embodiment.
[FIG. 16] FIG. 16 is a flowchart illustrating an example of the lesion identification process according to the third embodiment.

### Description of Embodiments

Hereinafter, embodiments of the present disclosure will be described with reference to the drawings.

### [First Embodiment]

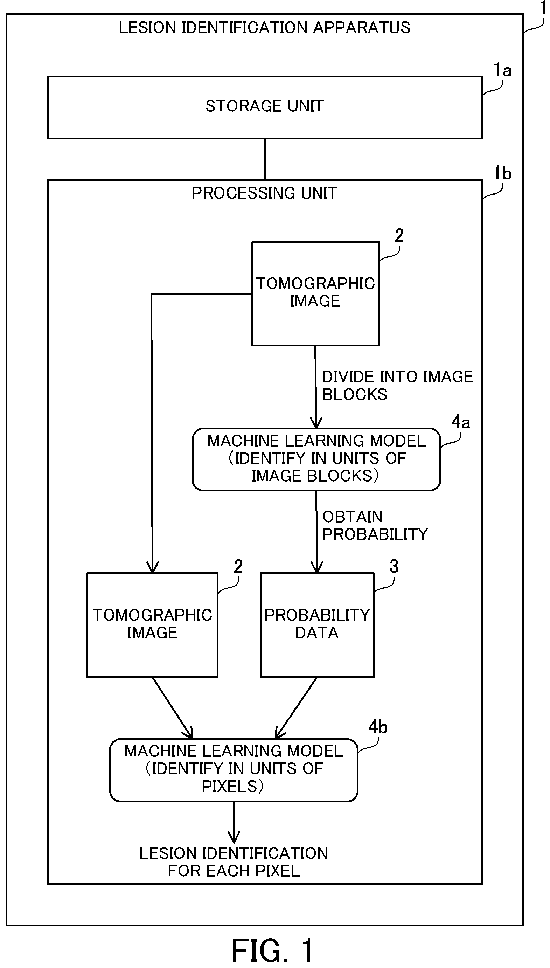

FIG. 1 illustrates a configuration example and a processing example of a lesion identification apparatus according to a first embodiment. A lesion identification apparatus 1 illustrated in FIG. 1 is an information processing apparatus that identifies, for image regions included in a tomographic image 2 obtained by imaging the inside of a human body, whether these image regions are regions of a specific lesion (lesion regions). The tomographic image 2 is a medical image captured by a CT apparatus, an MRI apparatus, or the like. In the following description, as an example, it is assumed that the tomographic image 2 is one of images captured at regular intervals in the height direction of the human body.

The lesion identification apparatus 1 includes a storage unit 1a and a processing unit 1b. The storage unit 1a is a storage area secured in a storage device, not illustrated, included in the lesion identification apparatus 1. The storage unit 1a stores, for example, model parameters indicating a machine learning model that performs a lesion identification process. The processing unit 1b is, for example, a processor. In this case, the following processing of the processing unit 1b is implemented by, for example, the processor executing a predetermined program. The processing unit 1b performs the following lesion identification process.

When the processing unit 1b receives the tomographic image 2, the processing unit 1b divides the tomographic image 2 into image blocks of a fixed size, and inputs each of the divided image blocks into a machine learning model 4a, thereby obtaining, for each image block, a probability that the image block is a lesion region. The machine learning model 4a is a trained model that is configured to identify, in units of image blocks of an input tomographic image, whether each image block is a lesion region. The machine learning model 4a is configured using, for example, a neural network. In this case, the processing unit 1b is able to obtain a value indicating a probability that an image block is a lesion region, from the final layer (output layer) of the neural network.

Next, the processing unit 1b generates probability data 3 including a probability for each pixel included in the tomographic image 2, based on the obtained probabilities. For example, for a certain image block of the tomographic image 2, the processing unit 1b assigns the probability acquired from the machine learning model 4a for the image block to all pixels included in the certain image block. The probability data 3 may be generated as, for example, a probability image (probability map) in which probability values are mapped to respective pixels of the tomographic image 2.

Next, the processing unit 1b identifies, for each pixel included in the tomographic image 2, whether that pixel is a lesion region, by inputting the tomographic image 2 and the probability data 3 to a machine learning model 4b. The machine learning model 4b is configured to identify, in units of pixels included in an input tomographic image, whether each pixel is a lesion region. For example, the machine learning model 4b is generated by machine learning using a plurality of tomographic images of one or more human bodies and probability data corresponding to the respective tomographic images as training input data and using labels for respective pixels of the plurality of tomographic images as ground truth data. The probability data for training is generated based on probabilities calculated using the first machine learning model 4a for respective image blocks obtained by dividing each of the plurality of tomographic images into image blocks of the above-described fixed size. Each label indicates whether each pixel of a corresponding one of the plurality of tomographic images is a lesion region.

Here, the machine learning model 4a identifies a lesion based on local image features in the tomographic image 2. Therefore, it may be said that the machine learning model 4a identifies a lesion based on simple image features such as brightness of an image. This machine learning model 4a is able to identify lesion regions with few missed detections, but may misidentify non-lesion regions as lesion regions. The probability data 3 may be regarded as a per-pixel identification result reflecting these advantages and disadvantages of the machine learning model 4a.

Now, as a comparative example, consider a machine learning model that, upon receiving a single tomographic image 2 as an input, identifies, in units of pixels of the tomographic image 2, whether each pixel is a lesion. This machine learning model is able to identify lesion regions based on smaller blocks of the tomographic image 2 than the machine learning model 4a. In addition, this machine learning model identifies lesion regions based on image features of the entire tomographic image 2, such as the shape of a lesion and a positional relationship with an organ, and is therefore less likely to misidentify non-lesion regions as lesion regions as compared with the machine learning model 4a. However, it is more likely to misidentify lesion regions as non-lesion regions as compared with the machine learning model 4a.

By contrast, the machine learning model 4b of the present embodiment receives not only the tomographic image 2 but also the probability data 3 as inputs, and thus is able to perform the lesion identification process that utilizes both the above advantages of the comparative example and the advantages of the machine learning model 4a. Specifically, the probability data 3, which reflects an identification result of the machine learning model 4a capable of identifying lesion regions with few missed detections, is input to the machine learning model 4b together with the tomographic image 2. Thus, in the lesion identification process performed by the machine learning model 4b, the likelihood that lesion regions are misidentified as non-lesion regions is reduced, which means mitigating the disadvantage of the comparative example. Thus, it is possible to identify lesion regions with high accuracy.

### [Second Embodiment]

The following describes a system that is capable of detecting a lesion region of a liver from a CT image.

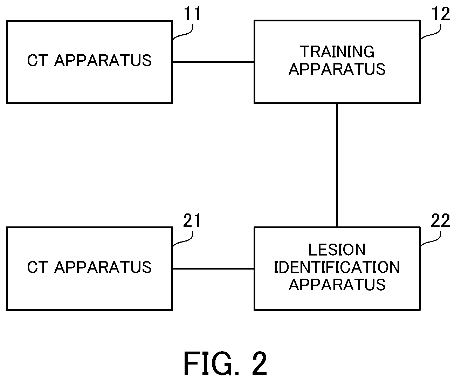

FIG. 2 illustrates a configuration example of a diagnosis support system according to a second embodiment. The diagnosis support system illustrated in FIG. 2 is a system that supports image diagnosis using CT imaging, and includes CT apparatuses 11 and 21, a training apparatus 12, and a lesion identification apparatus 22. The lesion identification apparatus 22 is an example of the lesion identification apparatus 1 illustrated in FIG. 1.

The CT apparatuses 11 and 21 capture CT images of a human body. In the present embodiment, the CT apparatuses 11 and 21 capture a predetermined number of tomographic images of axial planes in an abdominal region including the liver while changing a position (slice position) in the height direction (a direction perpendicular to the axial planes) of the human body at predetermined intervals.

The lesion identification apparatus 22 detects a lesion region from each tomographic image captured by the CT apparatus 21. In the present embodiment, it is assumed that a cancer region in the liver is detected as a lesion region. In addition, the lesion identification apparatus 22 detects the lesion region using a lesion identification model generated through machine learning. Further, the lesion identification apparatus 22 displays, for example, information indicating a result of identifying the lesion region on a display device. By doing so, the lesion identification apparatus 22 supports a user (for example, a radiologist) in image diagnosis.

The training apparatus 12 generates, by machine learning, the lesion identification model used by the lesion identification apparatus 22. The training apparatus 12 performs the machine learning for generating the lesion identification model using tomographic images captured by the CT apparatus 11 as training data. Data (model parameters) indicating the lesion identification model generated by the training apparatus 12 is loaded into the lesion identification apparatus 22 via, for example, a network or a portable recording medium.

Note that images captured by the same CT apparatus may be input to both the training apparatus 12 and the lesion identification apparatus 22. Alternatively, the training apparatus 12 may acquire the captured images via a recording medium or the like instead of directly acquiring the captured images from the CT apparatus. Furthermore, the training apparatus 12 and the lesion identification apparatus 22 may be implemented using the same information processing apparatus.

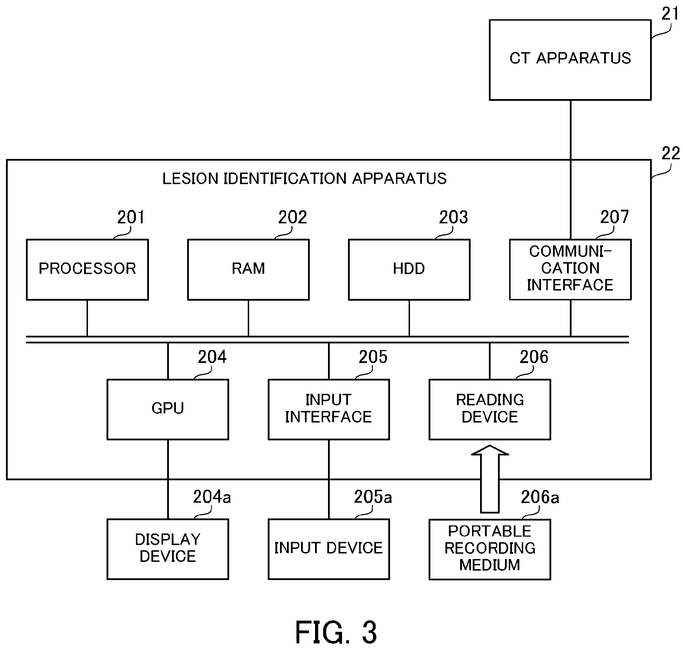

FIG. 3 illustrates an example of a hardware configuration of the lesion identification apparatus. The lesion identification apparatus 22 is implemented as, for example, a computer as illustrated in FIG. 3. As illustrated in FIG. 3, the lesion identification apparatus 22 includes a processor 201, a random access memory (RAM) 202, a hard disk drive (HDD) 203, a graphics processing unit (GPU) 204, an input interface 205, a reading device 206, and a communication interface 207.

The processor 201 (processor circuitry) comprehensively controls the entire lesion identification apparatus 22. The processor 201 is, for example, a central processing unit (CPU), a micro processing unit (MPU), a digital signal processor (DSP), an application specific integrated circuit (ASIC), or a programmable logic device (PLD). The processor 201 may be a combination of two or more elements selected from a CPU, an MPU, a DSP, an ASIC, and a PLD. The processor 201 is an example of the processing unit 1b illustrated in FIG. 1.

The RAM 202 is used as a main storage device of the lesion identification apparatus 22. The RAM 202 temporarily stores at least part of an operating system (OS) program and application programs to be executed by the processor 201. The RAM 202 also stores various data needed for the processor 201 to perform processing.

The HDD 203 is used as an auxiliary storage device of the lesion identification apparatus 22. The HDD 203 stores the OS program, application programs, and various data. As the auxiliary storage device, another type of non-volatile storage device such as a solid state drive (SSD) may be used.

A display device 204a is connected to the GPU 204. The GPU 204 displays images on the display device 204a in accordance with instructions from the processor 201. Examples of the display device 204a include a liquid crystal display and an organic electroluminescence (EL) display.

An input device 205a is connected to the input interface 205. The input interface 205 transmits signals received from the input device 205a to the processor 201. Examples of the input device 205a include a keyboard and a pointing device. Examples of the pointing device include a mouse, a touch panel, a tablet, a touch pad, and a track ball.

A portable recording medium 206a is attached to and detached from the reading device 206. The reading device 206 reads data from the portable recording medium 206a and transmits the data to the processor 201. Examples of the portable recording medium 206a include an optical disc and a semiconductor memory.

The communication interface 207 transmits and receives data to and from another apparatus such as the CT apparatus 21 via a network.

With the hardware configuration as described above, the processing functions of the lesion identification apparatus 22 are implemented. Note that the training apparatus 12 may also be implemented as a computer having a hardware configuration as illustrated in FIG. 3.

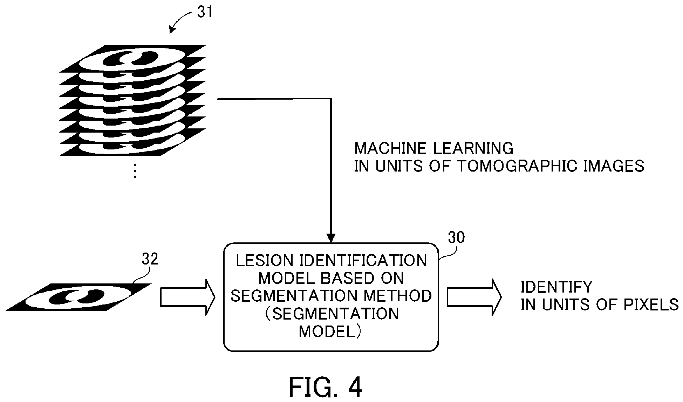

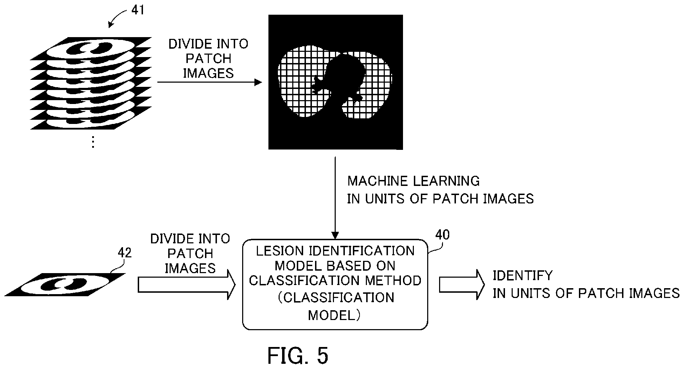

The following describes a comparative example of a lesion identification model generated through machine learning with reference to FIGS. 4 and 5.

FIG. 4 illustrates a first example of a lesion identification model. A lesion identification model 30 illustrated in FIG. 4 is a trained model that identifies a lesion using a segmentation method. Hereinafter, the lesion identification model 30 may be referred to as a "segmentation model 30". When the segmentation model 30 receives a tomographic image 32 as an input, the segmentation model 30 identifies, in units of pixels of the tomographic image 32, whether each pixel is a region of a specific lesion (in the present embodiment, whether each pixel represents liver cancer). Here, it is assumed that the tomographic image 32 is a tomographic image of an axial plane.

The segmentation model 30 is generated by machine learning (for example, deep learning) using a group of tomographic images 31 of axial planes as training data. A label indicating for each pixel whether the pixel is a lesion region is added to each tomographic image used as the training data, and the labels are used as ground truth data during the machine learning. The segmentation model 30 is generated by machine learning using such training data in units of tomographic images.

The segmentation model 30 may be a lesion identification model that identifies, for each pixel, one of three or more lesion classes including a class indicating a non-lesion. For example, in the case of identifying a lung shadow, the segmentation model 30 may identify, for each pixel, which one of six shadow classes the pixel corresponds to: ground-glass opacity, infiltrative shadow, honeycombing, emphysema, other shadows, and normal (not a shadow). In this case, a label indicating, for each pixel, which of the above-described shadow classes the pixel corresponds to is added to each tomographic image used as training data. The lesion identification process using this segmentation model 30 is effective for diagnosis of a diffuse lung disease, for example.

FIG. 5 illustrates a second example of the lesion identification model. A lesion identification model 40 illustrated in FIG. 5 is a trained model that identifies a lesion using a classification method. Hereinafter, this lesion identification model 40 may be referred to as a "classification model 40". When the classification model 40 receives a tomographic image 42 as an input, the classification model 40 divides the tomographic image 42 into patch images (image blocks) of a fixed size, and identifies, in units of divided patch images, whether each patch image is a region of a specific lesion (in the present embodiment, whether each patch image represents liver cancer). As with the tomographic image 32 of FIG. 4, it is assumed that the tomographic image 42 is a tomographic image of an axial plane.

The classification model 40 is generated by machine learning (for example, deep learning) using a group of tomographic images 41 of axial planes as training data. However, unlike the segmentation method in FIG. 4, each tomographic image used as the training data is divided into patch images as described above, and a label indicating whether a corresponding patch image is a lesion region is added to each divided patch image. Then, the classification model 40 is generated by machine learning using each patch image as teacher data and using the corresponding label as ground truth data in units of patch images.

The classification model 40 may be a lesion identification model that identifies, for each patch image, one of three or more lesion classes including a class indicating a non-lesion. For example, the classification model 40 may identify, for each patch image, which one of the above-described six shadow classes relating to the lung the patch image corresponds to. In this case, a label indicating, for each patch image, which of the above-described shadow classes the patch image corresponds to is added to each tomographic image used as training data.

In this way, the classification model 40 identifies a lesion in units of patch images each including a plurality of pixels. By contrast, the segmentation model 30 identifies a lesion in units of pixels, and is therefore able to identify a lesion based on smaller blocks of a tomographic image than the classification model 40.

In addition, the classification model 40 identifies a lesion based on simple image features such as brightness of an image. Therefore, the classification model 40 is able to identify, for example, a lesion appearing as a dark region in a tomographic image with few missed detections. However, the classification model 40 may misidentify, as a lesion, a non-lesion region that also appears as a dark region in a tomographic image. That is, the classification model 40 is characterized in that it achieves few missed detections but is likely to cause over-detections.

On the other hand, the segmentation model 30 identifies a lesion based on image features of an entire tomographic image, such as the shape of the lesion and its positional relationship with an organ. Therefore, the segmentation model 30 is able to accurately identify a lesion appearing as a dark region in a tomographic image with fewer errors, for example, as compared with the classification model 40 that identifies a lesion based on local image features within a patch image. However, the segmentation model 30 may misidentify a lesion appearing as a dark region in a tomographic image as a non-lesion. That is, the segmentation model 30 is characterized in that it achieves few over-detections of a lesion but is more likely to cause missed detections as compared with the classification model 40.

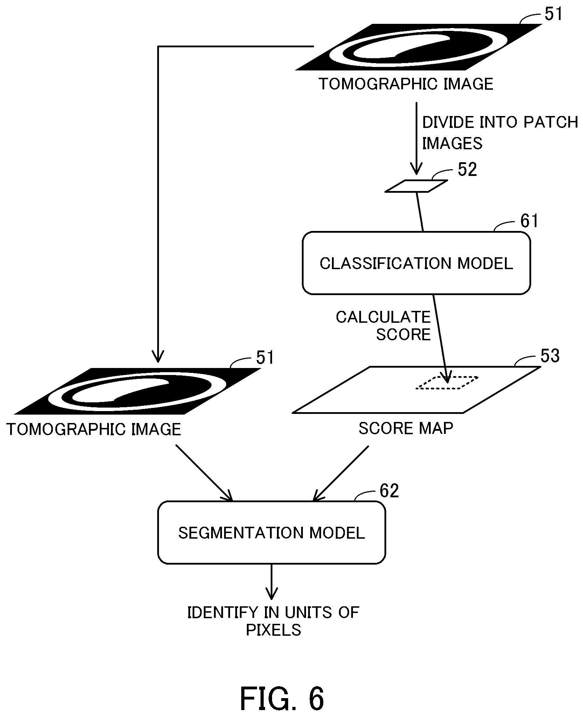

FIG. 6 illustrates a lesion identification process according to the second embodiment. In view of the above-described characteristics of the classification model and the segmentation model, the lesion identification apparatus 22 according to the present embodiment performs the lesion identification process using both the classification model and the segmentation model.

As illustrated in FIG. 6, the lesion identification apparatus 22 first divides an input tomographic image 51 into patch images 52 of a fixed size. The lesion identification apparatus 22 inputs each of the divided patch images 52 into a classification model 61 to perform the lesion identification process. As described with reference to FIG. 5, the classification model 61 is generated by machine learning using a group of tomographic images as training data, and is configured to identify, in units of patch images 52, whether each patch image 52 is a lesion.

By performing the lesion identification process using the classification model 61, the lesion identification apparatus 22 calculates, for each patch image 52, a score indicating a probability that the patch image 52 is a lesion. The classification model 61 is, for example, a machine learning model configured using a neural network. In this case, the scores are output from the final layer (output layer) of the neural network. As an example, each score takes a value between 0 and 1.

The lesion identification apparatus 22 generates a score map 53 using the scores calculated for the respective patch images 52. The score calculated for each patch image 52 is mapped to pixels corresponding to that patch image 52 in the area of the score map 53. The score map 53 is data in which a score is mapped to each pixel of the input tomographic image 51. Since the score is a probability as described above, the score map 53 may also be regarded as a probability image corresponding to the tomographic image 51.

Next, the lesion identification apparatus 22 inputs the input tomographic image 51 and the generated score map 53 into a segmentation model 62 to perform the lesion identification process on each pixel of the tomographic image 51. The segmentation model 62 is generated by machine learning using tomographic images and score maps as training input data and using labels indicating, for each pixel of the tomographic images, whether the pixel is a lesion as ground truth data. An identification result output from the segmentation model 62 for each pixel of the tomographic image 51 is a final identification result.

In the above processing, the score of each pixel in the score map 53 indicates a probability that the pixel is a lesion, calculated using the classification model 61. The scores in the score map 53 are likely to be high for lesion regions, and may also be likely to be high even for non-lesion regions. However, by performing the lesion identification process with the segmentation model 62 using both the score map 53 and the tomographic image 51 as input data, it becomes more likely that pixels having high scores despite being non-lesions are accurately identified as non-lesions.

In addition, as illustrated with reference to FIG. 4, the segmentation model that receives a tomographic image only as an input is likely to cause missed detections of a lesion. However, the segmentation model 62 receives, together with the tomographic image 51, the score map 53 reflecting the identification result of the classification model 61, which achieves few missed detections. Therefore, the segmentation model 62 reduces the likelihood that lesion pixels are misidentified as non-lesion pixels.

Therefore, the above-described processing of the lesion identification apparatus 22 makes it possible to identify, for each pixel of the tomographic image 51, whether that pixel is a lesion with high accuracy, compared to the case where only the classification model is used or the case where only the segmentation model is used.

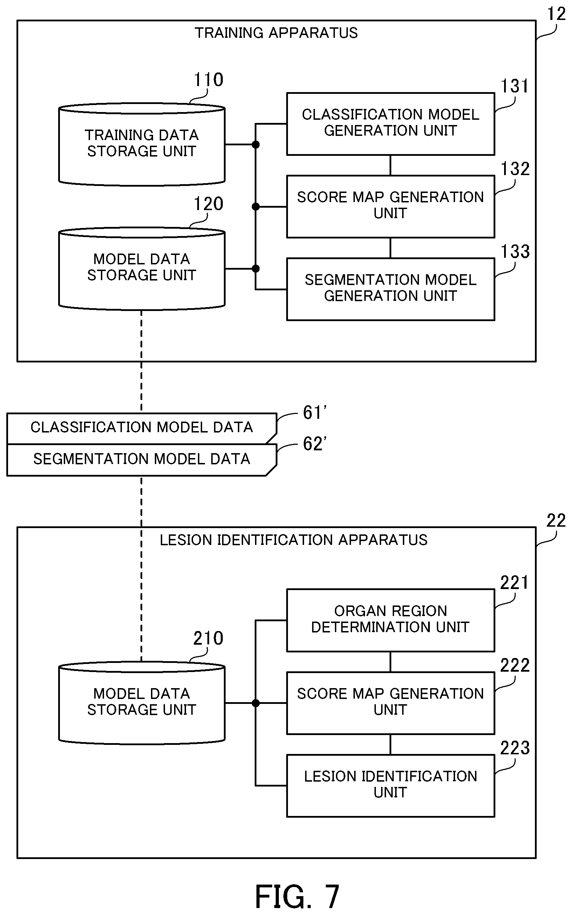

FIG. 7 illustrates a configuration example of processing functions included in each apparatus of the diagnosis support system.

First, the training apparatus 12 includes a training data storage unit 110, a model data storage unit 120, a classification model generation unit 131, a score map generation unit 132, and a segmentation model generation unit 133.

The training data storage unit 110 and the model data storage unit 120 are storage areas secured in a storage device, not illustrated, included in the training apparatus 12. The training data storage unit 110 stores training data used for generating the classification model 61 and the segmentation model 62 by machine learning. The model data storage unit 120 stores model data (model parameters) indicating the classification model 61 and the segmentation model 62 generated by machine learning.

The processing of the classification model generation unit 131, the score map generation unit 132, and the segmentation model generation unit 133 is implemented by, for example, a processor, not illustrated, included in the training apparatus 12 executing a predetermined application program.

The classification model generation unit 131 generates the classification model 61 by performing machine learning using training data for generating the classification model 61, the training data being stored in the training data storage unit 110. The classification model generation unit 131 stores classification model data 61' indicating the generated classification model 61 in the model data storage unit 120. In the case where the classification model 61 is configured using a neural network, the classification model data 61' includes weighting coefficients between nodes of the neural network.

The score map generation unit 132 generates a score map using the generated classification model 61, for each tomographic image included in training data for generating the segmentation model 62, the training data being stored in the training data storage unit 110.

The segmentation model generation unit 133 generates the segmentation model 62 by performing machine learning using the training data for generating the segmentation model 62 and the generated score maps, the training data being stored in the training data storage unit 110. The segmentation model generation unit 133 stores segmentation model data 62' indicating the generated segmentation model 62 in the model data storage unit 120. In the case where the segmentation model 62 is configured using a neural network, the segmentation model data 62' includes weighting coefficients between nodes of the neural network.

The classification model data 61' and the segmentation model data 62' generated by the training apparatus 12 are transferred to the lesion identification apparatus 22 via a network or a portable recording medium.

Next, the lesion identification apparatus 22 includes a model data storage unit 210, an organ region determination unit 221, a score map generation unit 222, and a lesion identification unit 223.

The model data storage unit 210 is a storage area secured in a storage device included in the lesion identification apparatus 22, such as the RAM 202 or the HDD 203. The model data storage unit 210 stores data on an organ region identification model, and the classification model data 61' and the segmentation model data 62' received from the lesion identification apparatus 22. The organ region identification model is a trained model that determines a region of an organ (liver in the present embodiment) from a tomographic image set, and is generated in advance by machine learning.

The processing of the organ region determination unit 221, the score map generation unit 222, and the lesion identification unit 223 is implemented by, for example, the processor 201 executing a predetermined application program.

The organ region determination unit 221 inputs an input tomographic image set into the organ region identification model to determine an organ region from the tomographic image set. Through this process, tomographic images that include the organ region are extracted from the tomographic image set, and the organ region is specified in the extracted tomographic images.

The score map generation unit 222 inputs the tomographic images including the organ region into the classification model 61 based on the classification model data 61' to calculate a score for each patch image. The score map generation unit 222 generates a score map corresponding to each tomographic image based on the calculated scores.

The lesion identification unit 223 inputs the tomographic images including the organ region and the corresponding score maps into the segmentation model 62 based on the segmentation model data 62' to perform the lesion identification process, and outputs a lesion identification result for each pixel.

Note that the classification model generation unit 131, the score map generation unit 132, the segmentation model generation unit 133, the organ region determination unit 221, the score map generation unit 222, and the lesion identification unit 223 may be provided in the same apparatus. That is, the training process for the classification model 61 and the segmentation model 62 and the lesion identification process using the classification model 61 and the segmentation model 62 may be performed by the same apparatus.

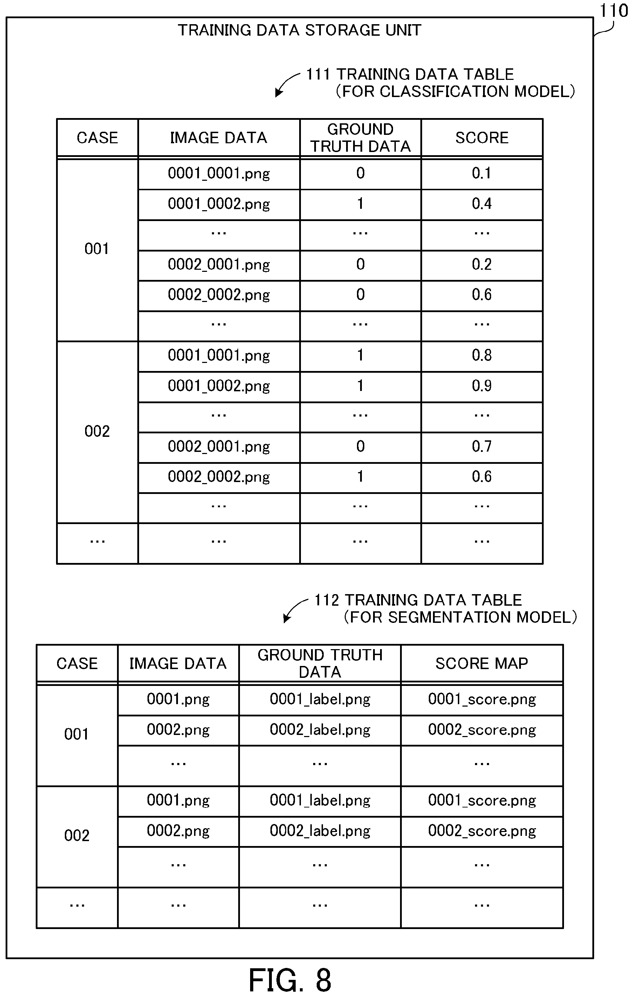

FIG. 8 illustrates an example of data stored in the training data storage unit. The training data storage unit 110 stores training data tables 111 and 112.

The training data table 111 stores training data used for generating the classification model 61. The training data table 111 contains a plurality of sets of training data with respect to each of a plurality of cases, each set including image data and ground truth data serving as input data.

The image data is data of a patch image obtained by dividing a tomographic image into a predetermined size. A group of image data corresponding to a case is a group of image data of patch images derived from tomographic images, the tomographic images being acquired by a single imaging session for a single person. For each patch image, the tomographic image from which the patch image is derived is identifiable. In the example of FIG. 8, the first four digits of the file names of the image data of the patch images are the same as the file name of the image data of the tomographic image. The ground truth data is a label indicating whether the corresponding patch image is a lesion.

The training data table 111 further contains scores calculated using the generated classification model 61. Each score is registered in association with the image data of a corresponding patch image from which that score is calculated. As described above, each score takes a value between 0 and 1.

The training data table 112 stores training data used for generating the segmentation model 62. The training data table 112 contains a plurality of sets of training data with respect to each of a plurality of cases, each set including image data, ground truth data, and a score map.

The image data and the score map are used as input data during machine learning. The image data is data of a tomographic image, and a group of image data corresponding to a case is a group of image data of tomographic images acquired by a single imaging session for a single person. These tomographic images are the tomographic images from which the patch images registered in the training data table 111 are derived. The ground truth data is data in which each pixel of a corresponding tomographic image is associated with a label indicating whether the pixel is a lesion. The score map is generated for each tomographic image based on the scores registered in the training data table 111, and is registered in the training data table 112.

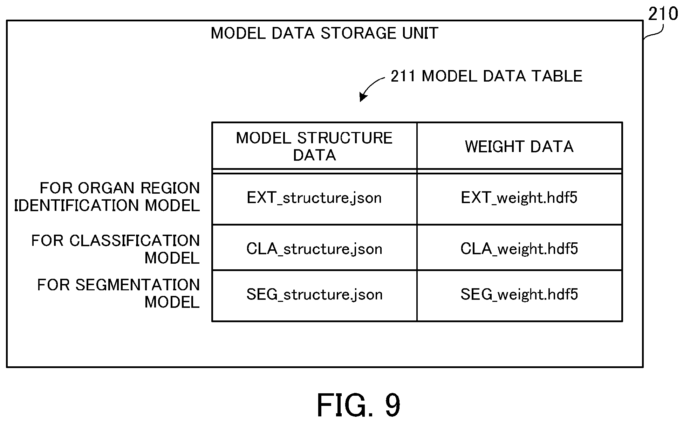

FIG. 9 illustrates an example of data stored in the model data storage unit. The model data storage unit 210 stores a model data table 211. The model data table 211 stores model structure data and weight data for each of the organ region identification model, the classification model 61, and the segmentation model 62.

The model structure data indicates the type of a machine learning model to be used and the configuration of a neural network. In practice, the file name of a file describing such information may be registered in the model data table 211. The weight data indicates weighting coefficients between nodes in the neural network of the machine learning model. In practice, the file name of a file describing such weighting coefficients may be registered in the model data table 211.

The model structure data and the weight data for the organ region identification model are registered in the model data table 211 in advance. The model structure data and the weight data for the classification model 61 and the segmentation model 62 are generated by the training apparatus 12 and registered in the model data table 211. In this case, the model structure data and the weight data for the classification model 61 correspond to the classification model data 61' described above, and the model structure data and the weight data for the segmentation model 62 correspond to the segmentation model data 62' described above. Note that the model structure data for the classification model 61 and the segmentation model 62 may be registered in the model data table 211 in advance.

Note that the model data storage unit 120 of the training apparatus 12 stores a model data table in which at least the model structure data and the weight data for the classification model 61 and the segmentation model 62 are registered.

Next, the process of the training apparatus 12 will be described with reference to a flowchart.

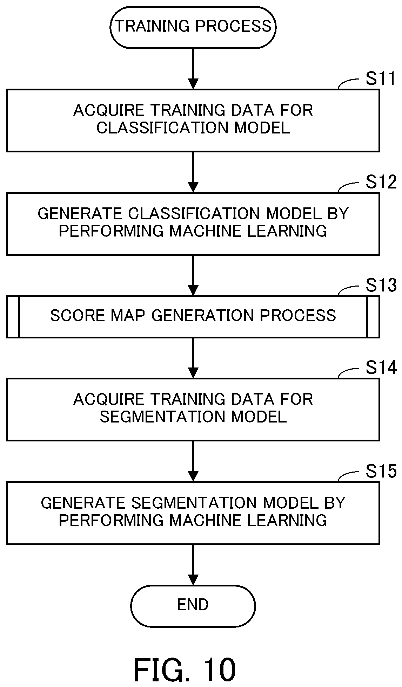

FIG. 10 is a flowchart illustrating an example of a training process performed by the training apparatus.

[Step S11] The classification model generation unit 131 acquires training data from the training data table 111.

[Step S12] The classification model generation unit 131 generates the classification model 61 by performing machine learning using the acquired training data. In this step, the machine learning is performed using each patch image included in the training data as training input data and using ground truth data corresponding to that patch image as training ground truth data. As a result, the classification model data 61' is generated and registered in the model data storage unit 120.

[Step S13] The score map generation unit 132 calculates a score for each patch image by inputting each patch image registered in the training data table 111 into the generated classification model 61. The calculated scores are registered in association with the patch images in the training data table 111. Then, a score map is generated for each tomographic image based on the registered scores.

[Step S14] The segmentation model generation unit 133 acquires training data from the training data table 112.

[Step S15] The segmentation model generation unit 133 generates the segmentation model 62 by performing machine learning using the acquired training data and the generated score maps. In this step, the machine learning is performed using each tomographic image included in the training data and the score map corresponding to that tomographic image as training input data, and using ground truth data corresponding to each tomographic image as training ground truth data. As a result, the segmentation model data 62' is generated and registered in the model data storage unit 120.

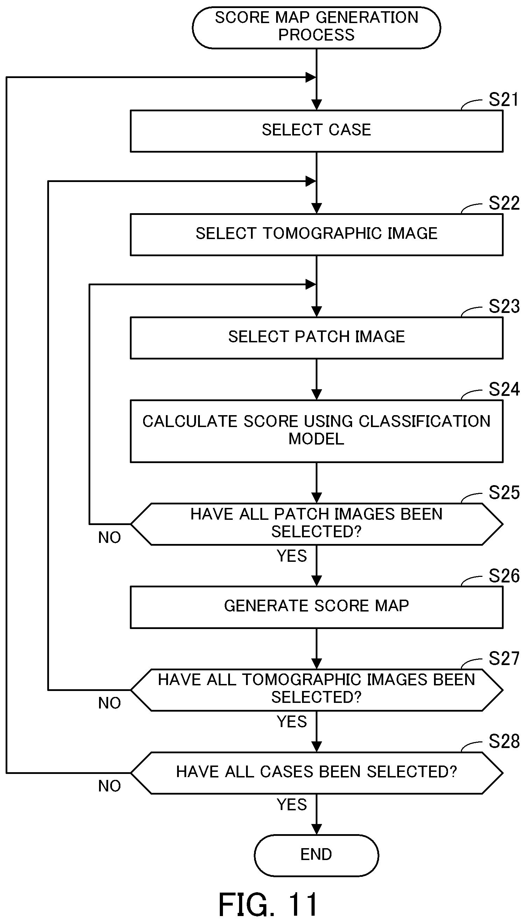

FIG. 11 is a flowchart illustrating an example of a score map generation process. The process of FIG. 11 corresponds to step S13 of FIG. 10.

[Step S21] The score map generation unit 132 selects one case from the training data table 111.

[Step S22] The score map generation unit 132 selects one tomographic image included in the selected case.

[Step S23] The score map generation unit 132 selects one patch image included in the selected tomographic image.

[Step S24] The score map generation unit 132 calculates a score by inputting the selected patch image into the classification model 61. The calculated score is registered in a record corresponding to the selected patch image in the training data table 111.

[Step S25] The score map generation unit 132 determines whether all patch images included in the selected tomographic image have been selected. If there is any unselected patch image, the process proceeds to step S23, and one unselected patch image is selected. On the other hand, if all the patch images have been selected, the process proceeds to step S26.

[Step S26] The score map generation unit 132 generates a score map corresponding to the selected tomographic image. In this step, the score calculated for each patch image is mapped to all pixels within a region corresponding to that patch image in the tomographic image.

[Step S27] The score map generation unit 132 determines whether all tomographic images included in the selected case have been selected. If there is any unselected tomographic image, the process proceeds to step S22, and one unselected tomographic image is selected. On the other hand, if all the tomographic images have been selected, the process proceeds to step S28.

[Step S28] The score map generation unit 132 determines whether all cases registered in the training data table 111 have been selected. If there is any unselected case, the process proceeds to step S21, and one unselected case is selected. On the other hand, if all the cases have been selected, the score map generation process is completed.

Next, the process of the lesion identification apparatus 22 will be described with reference to a flowchart.

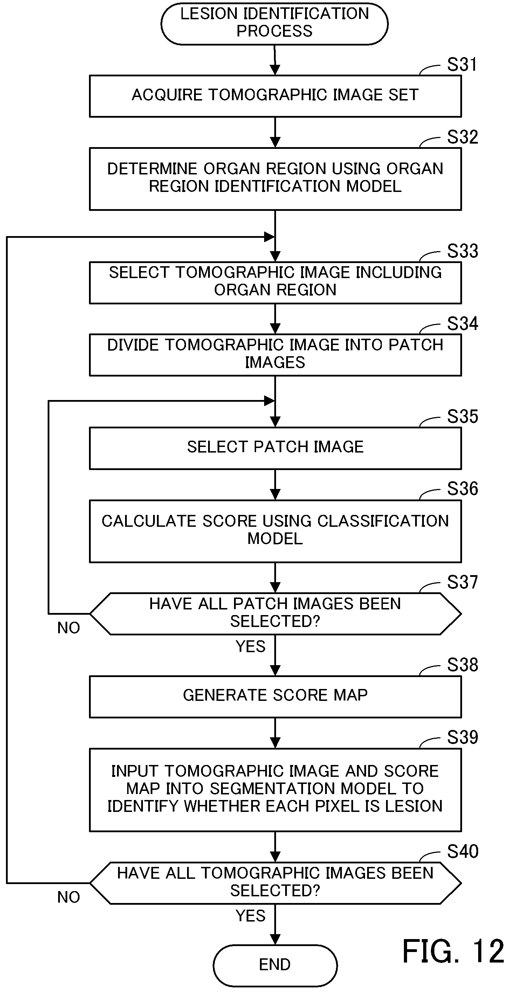

FIG. 12 is a flowchart illustrating an example of a lesion identification process performed by the lesion identification apparatus.

[Step S31] The lesion identification apparatus 22 acquires, from the CT apparatus 21, a tomographic image set obtained by imaging a diagnosis target person.

[Step S32] The organ region determination unit 221 inputs the acquired tomographic image set into the organ region identification model to determine an organ region from the tomographic image set. Through this step, tomographic images including the organ region are extracted from the tomographic image set, and the organ region is specified in the extracted tomographic images.

[Step S33] The score map generation unit 222 selects one tomographic image including the organ region from the tomographic image set.

[Step S34] The score map generation unit 222 divides the selected tomographic image into patch images of a predetermined size.

[Step S35] The score map generation unit 222 selects one of the patch images obtained by the division. In this step, a patch image including the organ region is selected.

[Step S36] The score map generation unit 222 calculates a score by inputting the selected patch image into the classification model 61.

[Step S37] The score map generation unit 222 determines whether all patch images included in the selected tomographic image have been selected. If there is any unselected patch image, the process proceeds to step S35, and one unselected patch image is selected. On the other hand, if all the patch images have been selected, the process proceeds to step S38.

[Step S38] The score map generation unit 222 generates a score map corresponding to the selected tomographic image. In this step, the score calculated for each patch image is mapped to all pixels within a region corresponding to that patch image in the tomographic image.

[Step S39] The lesion identification unit 223 inputs the selected tomographic image and the generated score map into the segmentation model 62, to identify, for each pixel of the tomographic image, whether that pixel is a lesion.

[Step S40] The lesion identification unit 223 determines whether all tomographic images including the organ region have been selected. If there is any unselected tomographic image, the process proceeds to step S33, and one unselected tomographic image is selected. On the other hand, if all the tomographic images have been selected, the lesion identification process is completed.

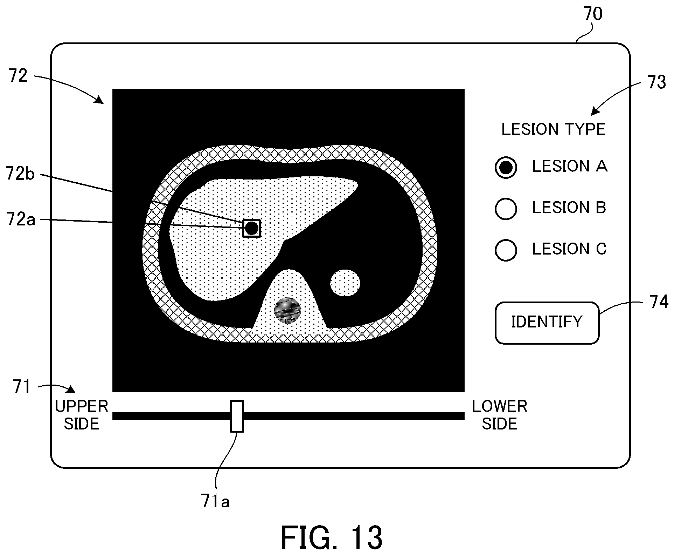

FIG. 13 illustrates an example of a screen that displays a lesion identification result. The lesion identification apparatus 22 is able to present a lesion identification result to a user by, for example, displaying an identification result screen 70 as illustrated in FIG. 13 on the display device 204a. The identification result screen 70 includes a slice selection area 71, a tomographic image display area 72, a lesion type selection area 73, and an identification button 74.

In the slice selection area 71, the slice plane of a tomographic image to be displayed in the tomographic image display area 72 may be selected by moving a handle 71a on a slider. The tomographic image display area 72 displays a tomographic image corresponding to a slice plane selected via the slice selection area 71. In this tomographic image, a lesion region is displayed based on a lesion identification result corresponding to the same slice plane. In FIG. 13, as an example, a rectangle 72b surrounding a lesion region 72a is superimposed and displayed on the tomographic image. As another display method, the lesion region 72a may be displayed in a specific color. The lesion region 72a is a region of a group of pixels identified as a lesion.

The lesion type selection area 73 allows a user to select a lesion type for which an identification result is to be displayed. Here, for example, it is assumed that the classification model 61 and the segmentation model 62 are generated for each lesion type. In this case, a machine learning model to be used for lesion identification is selected via the lesion type selection area 73. When a lesion type is selected in the lesion type selection area 73 and the identification button 74 is pressed, an identification result obtained using the selected machine learning model is displayed in the tomographic image display area 72.

### [Third Embodiment]

A third embodiment will now be described as a modification of a part of the processing performed in the diagnosis support system of the second embodiment. The third embodiment uses a plurality of score maps generated using patch images having different sizes.

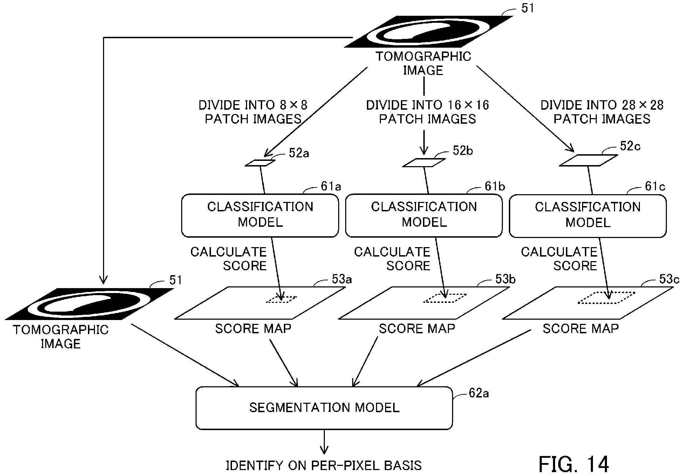

FIG. 14 illustrates a lesion identification process according to the third embodiment.

The lesion identification apparatus 22 of the present embodiment first divides an input tomographic image 51 into patch images having different sizes. In the example of FIG. 14, the tomographic image 51 is divided into patch images 52a each having a size of 8 pixels × 8 pixels. The tomographic image 51 is also divided into patch images 52b each having a size of 16 pixels × 16 pixels. Further, the tomographic image 51 is divided into patch images 52c each having a size of 28 pixels × 28 pixels.

The lesion identification apparatus 22 inputs each of the divided patch images 52a into a classification model 61a to calculate a score for each patch image 52a. The classification model 61a is a machine learning model trained using patch images of 8 pixels × 8 pixels as training data. The lesion identification apparatus 22 generates a score map 53a using the scores calculated for the respective patch images 52a using the classification model 61a.

The lesion identification apparatus 22 inputs each of the divided patch images 52b into a classification model 61b to calculate a score for each patch image 52b. The classification model 61b is a machine learning model trained using patch images of 16 pixels × 16 pixels as training data. The lesion identification apparatus 22 generates a score map 53b using the scores calculated for the respective patch images 52b using the classification model 61b.

Further, the lesion identification apparatus 22 inputs each of the divided patch images 52c into a classification model 61c to calculate a score for each patch image 52c. The classification model 61c is a machine learning model trained using patch images of 28 pixels × 28 pixels as training data. The lesion identification apparatus 22 generates a score map 53c using the scores calculated for the respective patch images 52c using the classification model 61c.

Then, the lesion identification apparatus 22 inputs the input tomographic image 51 and the generated score maps 53a to 53c into a segmentation model 62a to perform a lesion identification process on a per-pixel basis for the tomographic image 51. The segmentation model 62a is generated by machine learning using, as training input data, a tomographic image, a score map generated using the classification model 61a based on patch images of 8 pixels × 8 pixels, a score map generated using the classification model 61b based on patch images of 16 pixels × 16 pixels, and a score map generated using the classification model 61c based on patch images of 28 pixels × 28 pixels, and using, as ground truth data, a label indicating, for each pixel of the tomographic image, whether the pixel is a lesion. An identification result output from the segmentation model 62a for each pixel of the tomographic image 51 is a final identification result.

According to the above process, a lesion may be identified based on features in local regions of various sizes, which improves lesion identification accuracy. For example, a lesion whose size varies across patch images having different sizes may be accurately identified without omission.

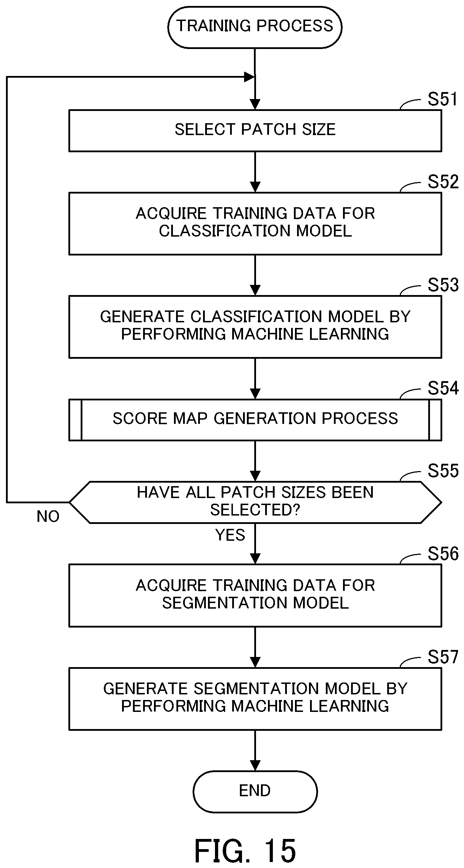

FIG. 15 is a flowchart illustrating an example of the training process according to the third embodiment.

[Step S51] The classification model generation unit 131 selects a patch size.

[Step S52] Training data tables 111 corresponding to respective patch sizes are stored in the training data storage unit 110. The classification model generation unit 131 identifies the training data table 111 corresponding to the selected patch size from the training data storage unit 110, and acquires training data from the identified training data table 111.

[Step S53] The classification model generation unit 131 generates the classification model 61 corresponding to the selected patch size by performing machine learning using the acquired training data. In this step, the machine learning is performed using each patch image included in the training data as training input data and using ground truth data corresponding to that patch image as training ground truth data. As a result, the classification model data 61' corresponding to the selected patch size is generated and registered in the model data storage unit 120.

[Step S54] The score map generation unit 132 calculates a score for each patch image by inputting each patch image registered in the identified training data table 111 into the generated classification model 61. The calculated scores are registered in association with the corresponding patch images in the training data table 111. Then, a score map is generated for each tomographic image based on the registered scores. In step S54, the process illustrated in FIG. 11 is performed for each score size.

[Step S55] The score map generation unit 132 determines whether all patch sizes have been selected. If there is any unselected patch size, the process proceeds to step S51, and one unselected patch size is selected. On the other hand, if all the patch sizes have been selected, the process proceeds to step S56.

[Step S56] The segmentation model generation unit 133 acquires training data from the training data table 112.

[Step S57] The segmentation model generation unit 133 generates the segmentation model 62 by performing machine learning using the acquired training data and the generated score maps. In this step, the machine learning is performed using each tomographic image included in the training data and the score maps corresponding to that tomographic image for the respective patch sizes as training input data, and using ground truth data corresponding to that tomographic image as training ground truth data. As a result, data indicating the segmentation model 62 is generated and registered in the model data storage unit 120.

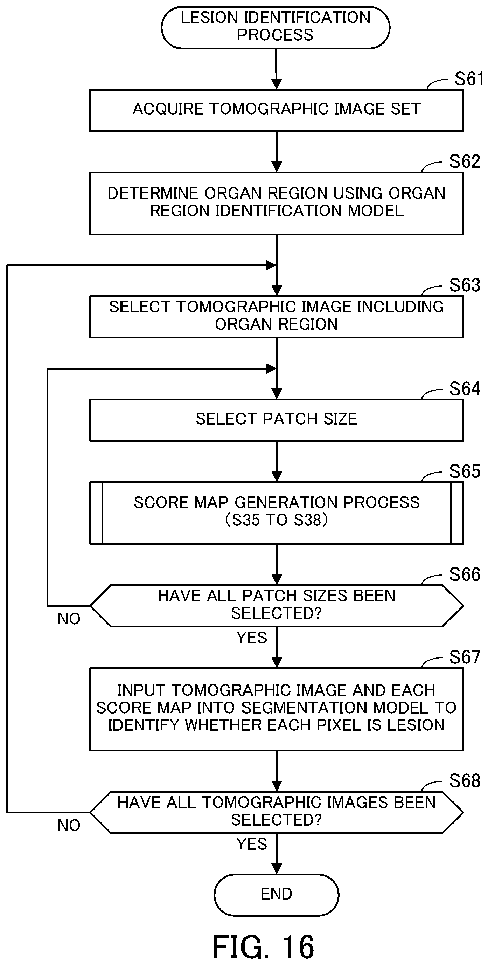

FIG. 16 is a flowchart illustrating an example of the lesion identification process according to the third embodiment.

[Step S61] The lesion identification apparatus 22 acquires, from the CT apparatus 21, a tomographic image set obtained by imaging a diagnosis target person.

[Step S62] The organ region determination unit 221 inputs the acquired tomographic image set into the organ region identification model to determine an organ region from the tomographic image set. Through this step, tomographic images including the organ region are extracted from the tomographic image set, and the organ region is specified in the extracted tomographic images.

[Step S63] The score map generation unit 222 selects one tomographic image including the organ region from the tomographic image set.

[Step S64] The score map generation unit 222 selects a patch size.

[Step S65] The score map generation unit 222 generates a score map corresponding to the selected patch size. This step is executed by applying the selected patch size to steps S35 to S38 of FIG. 12. In step S36, the classification model 61 corresponding to the selected patch size is used.

[Step S66] The score map generation unit 222 determines whether all patch sizes have been selected. If there is any unselected patch size, the process proceeds to step S64, and one unselected patch size is selected. On the other hand, if all the patch sizes have been selected, the process proceeds to step S67.

[Step S67] The lesion identification unit 223 inputs the selected tomographic image and the generated score maps for the respective patch sizes into the segmentation model 62a to identify, for each pixel of the tomographic image, whether the pixel is a lesion.

[Step S68] The lesion identification unit 223 determines whether all tomographic images including the organ region have been selected. If there is any unselected tomographic image, the process proceeds to step S63, and one unselected tomographic image is selected. On the other hand, if all the tomographic images have been selected, the lesion identification process is completed.

The processing functions of the apparatuses (for example, the lesion identification apparatuses 1 and 22 and the training apparatus 12) described in the above embodiments may be implemented by a computer. In this case, a program describing the processing contents of the functions to be executed by each apparatus is provided, and the processing functions are implemented on the computer by executing the program on the computer. The program describing the processing contents may be recorded on a computer-readable recording medium. Examples of the computer-readable recording medium include magnetic storage devices, optical discs, and semiconductor memories. Examples of the magnetic storage devices include a hard disk drive (HDD) and a magnetic tape. Examples of the optical discs include a compact disc (CD), a digital versatile disc (DVD), and a Blu-ray disc (BD, registered trademark).

To distribute the program, for example, portable recording media such as DVDs or CDs on which the program is recorded are sold. Alternatively, the program may be stored in a storage device of a server computer and transferred from the server computer to other computers via a network.

A computer that executes the program stores, for example, the program recorded on a portable recording medium or the program transferred from the server computer in its local storage device. Then, the computer reads the program from the local storage device and performs processing according to the program. The computer may also read the program directly from the portable recording medium and perform processing according to the program. In addition, each time the program is transferred from the server computer connected via the network, the computer may sequentially perform processing according to the received program.

The above description is merely indicative of the principles of the embodiments. A wide variety of modifications and changes may also be made by those skilled in the art. The embodiments are not limited to the precise configurations and example applications indicated and described above, and all appropriate modifications and equivalents are regarded as falling within the scope of the embodiments as defined by the appended patent claims and their equivalents.

### Reference Signs List

1 Lesion identification apparatus
1a Storage unit
1b Processing unit
2 Tomographic image
3 Probability data
4a, 4b Machine learning model

## Claims

1. A lesion identification method executed by a computer, the lesion identification method comprising:
acquiring a first tomographic image obtained by imaging an inside of a first human body;
obtaining, for each first image block included in the first tomographic image, a first probability that each first image block is a region of a specific lesion, by inputting the first tomographic image into a first machine learning model, the first machine learning model being configured to identify, in units of first image blocks obtained by dividing an input tomographic image into a predetermined size, whether each of the first image blocks of the input tomographic image is a region of the specific lesion;
generating, based on the obtained first probability, first probability data including a second probability assigned to each pixel included in the first tomographic image; and
identifying, for each pixel included in the first tomographic image, whether each pixel is a region of the specific lesion, by inputting the first tomographic image and the first probability data into a second machine learning model, the second machine learning model being configured to identify, in units of pixels included in the input tomographic image, whether each of the pixels included in the input tomographic image is a region of the specific lesion.

2. The lesion identification method according to claim 1, further comprising:
generating the second machine learning model by performing machine learning using, as training input data, a plurality of tomographic images obtained by imaging insides of one or more second human bodies and second probability data including a third probability calculated for each pixel of the plurality of tomographic images, and using, as ground truth data, a label indicating, for each pixel of the plurality of tomographic images, whether each pixel is a region of the specific lesion, the third probability being calculated based on second probability calculated using the first machine learning model for each second image block obtained by dividing each of the plurality of tomographic images into the predetermined size.

3. The lesion identification method according to claim 1, wherein:
the obtaining of the first probability includes obtaining the first probability using each of a plurality of first machine learning models including the first machine learning model, by inputting the first tomographic image to each of the plurality of first machine learning models, the plurality of first machine learning models each being configured to identify, in units of image blocks having a different size, whether each of the image blocks is a region of the specific lesion;
the generating of the first probability data includes generating a plurality of first probability data corresponding respectively to the plurality of first machine learning models, the plurality of first probability data each being generated based on the first probability obtained using a corresponding one of the plurality of first machine learning models; and
the identifying includes identifying, for each pixel included in the first tomographic image, whether each pixel is a region of the specific lesion, by inputting the first tomographic image and the plurality of probability data into the second machine learning model.

4. A lesion identification program that causes a computer to perform a process comprising:
acquiring a first tomographic image obtained by imaging an inside of a first human body;
obtaining, for each first image block included in the first tomographic image, a first probability that each first image block is a region of a specific lesion, by inputting the first tomographic image into a first machine learning model, the first machine learning model being configured to identify, in units of first image blocks obtained by dividing an input tomographic image into a predetermined size, whether each of the first image blocks of the input tomographic image is a region of the specific lesion;
generating, based on the obtained first probability, probability data including a second probability for each pixel included in the first tomographic image; and
identifying, for each pixel included in the first tomographic image, whether each pixel is a region of the specific lesion, by inputting the first tomographic image and the probability data into a second machine learning model, the second machine learning model being configured to identify, in units of pixels included in the input tomographic image, whether each of the pixels included in the input tomographic image is a region of the specific lesion.
